# EUROPÄISCHE PATENTANMELDUNG

(11) **EP 1 378 849 A1**
(43) Veröffentlichungstag der Anmeldung: **07.01.2004**
(21) Anmeldenummer: 02014701.3
(22) Anmeldetag: 03.07.2002
(51) Int. Cl.: G06F 19/00

(54) **Verfahren und System zur Unterstützung der Therapieplanung in der Rehabilitation**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisenmann, Uwe, 91052 Erlangen (DE); Richter, Niels, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Unterstützung der Therapieplanung in der Rehabilitation eines Patienten, bei dem Patientendaten eines Patienten erfasst werden, die Messwerte von Messgrößen über die Durchführung und/oder zur Quantifizierung von Zwischenergebnissen eines vom Patienten absolvierten Trainingsprogramms umfassen. Weiterhin wird eine erste Datenbank (11) bereitgestellt, die Regeln zur Verknüpfung von Patientendaten mit Vorschlägen zur Modifikation von Trainingsprogrammen enthält. Durch eine erste oder zweite Datenverarbeitungsstation (10) werden durch Rückgriff auf die erste Datenbank (11) ein oder mehrere Vorschläge zur Modifikation oder Beibehaltung des vom Patienten absolvierten Trainingsprogramms automatisch generiert und an der ersten oder einer weiteren Datenverarbeitungsstation (30) ausgegeben. Das vorliegende Verfahren sowie das zugehörige System verringern die Arbeits- und Zeitbelastung eines betreuenden Arztes oder Therapeuten bei der Modifikation eines Trainingsprogramms während einer Rehabilitation.

## Beschreibung

Die vorliegende Erfindung betrifft ein Verfahren sowie ein System zur Unterstützung der Therapieplanung in der Rehabilitation eines Patienten, bei dem das Trainingsprogramm des Patienten überwacht und in Abhängigkeit von der Durchführung und/oder von Zwischenergebnissen des Trainings modifiziert wird.

Eine wesentliche Komponente der Rehabilitation ist das Eigentraining des Patienten zur Verbesserung rehabilitationsrelevanter Fähigkeiten. Bei diesem Training handelt es sich in Abhängigkeit vom jeweiligen Fähigkeitsdefizit des Patienten um ein Training motorischer Fähigkeiten, wie bspw. Kraft, Beweglichkeit oder Gleichgewichtsfähigkeit, und/oder kognitiver Fähigkeiten, wie bspw. Aufmerksamkeit oder Gedächtnis. So werden in der orthopädischen Rehabilitation, bspw. nach Knochenfrakturen, und in der kardiologischen Rehabilitation, bspw. nach Herzinfarkt, vorrangig motorische Fähigkeiten trainiert. In der neurologischen Rehabilitation, bspw. nach Hirninfarkt/Hirnblutung, werden dagegen vorrangig kognitive Fähigkeiten trainiert. Wenn motorische und kognitive Funktionen des Patienten beeinträchtigt sind, ist ein kombiniertes Training motorischer und kognitiver Fähigkeiten indiziert. Diese Trainingsform wird unter anderem in der geriatrischen Rehabilitation, bspw. bei Morbus Alzheimer, und auch in der neurologischen Rehabilitation bei komplexen Schädigungen, wie bspw. Schlaganfall mit Halbseitenlähmung, favorisiert.

Während der Dauer eines Trainingsprogramms, die sich über mehrere Wochen bis hin zu Jahren erstrecken kann, muss der Patient kontinuierlich überwacht und bei erheblichen Abweichungen der Entwicklung des Patienten vom Trainingsziel bzw. von entsprechenden Trainingsteilzielen ggf. das Training modifiziert werden. Eine derartige Modifikation des Trainingsprogramms kann auch erforderlich werden, wenn beim Patienten im Verlauf des Trainings Krankheiten auftreten, aufgrund derer der Patient bestimmte Übungen des Trainingsprogramms nicht mehr durchführen kann.

Der Arzt oder Therapeut erhält während des Trainingsprogramms daher in der Regel ein entsprechendes Feedback, insbesondere in Form von Messgrößen, die die momentane Leistung des Patienten bei der Durchführung einzelner Übungen des Trainingsprogramms oder das Ergebnis entsprechender Zwischentests widerspiegeln. Weitere Informationsquellen sind bspw. Einträge in ein Patiententagebuch oder ein direktes Gespräch mit dem Patienten. Der Arzt oder Therapeut bewertet dann die Patientendaten einschließlich der übermittelten Messgrößen intellektuell, um daraus vorteilhafte oder notwendige Modifikationen des verschriebenen Trainingsprogramms abzuleiten. Dieses Vorgehen ist jedoch zeit- und arbeitsintensiv.

Aus der noch nicht.veröffentlichten DE 10147471.7 ist ein Verfahren und System zur Unterstützung des Arztes oder Therapeuten bei der Erstellung eines Therapieplanes bekannt, bei dem Patientendaten aus einer elektronischen Patientenakte einem computerbasierten Expertensystem zugeführt werden, das den erstellten Therapieplan auf Basis dieser Patientendaten beispielsweise auf Kontraindikationen überprüft. Auf diese Weise können Fehler bei der Erstellung des Therapieplanes vermieden werden. Der Zeit- und Arbeitsaufwand bei der Modifikation des Trainingsplanes während der Therapie wird durch dieses Verfahren bzw. System jedoch nicht verringert.

Ausgehend von dieser Situation besteht die Aufgabe der vorliegenden Erfindung darin, ein Verfahren sowie ein System zur Unterstützung der Therapieplanung in der Rehabilitation eines Patienten anzugeben, das den Zeit- und Arbeitsaufwand für den zuständigen Arzt oder Therapeuten verringert.

Die Aufgabe wird mit dem Verfahren sowie dem System gemäß den Patentansprüchen 1 und 18 gelöst. Vorteilhafte Ausgestaltungen des Verfahrens bzw. des Systems sind Gegenstand der Unteransprüche oder lassen sich aus der nachfolgenden Beschreibung und den Ausführungsbeispielen entnehmen.

Bei dem vorliegenden Verfahren zur Unterstützung der Therapieplanung in der Rehabilitation eines Patienten werden Patientendaten des Patienten erfasst, die Messwerte von Messgrößen über die Durchführung und/oder zur Quantifizierung von Zwischenergebnissen eines vom Patienten absolvierten Trainingsprogramms umfassen. Unter Zwischenergebnis ist hierbei der Trainingserfolg nach einzelnen Trainingseinheiten zu verstehen. Die Messwerte können dabei selbstverständlich auch aus aus einzelnen Messdaten mit Hilfe einer vorgegebenen Berechnungsvorschrift zusammengefasste Werte sein. Weiterhin wird eine erste Datenbank bereitgestellt, die eine Vielzahl an Regeln zur Verknüpfung von Patientendaten mit Vorschlägen zur Modifikation von Trainingsprogrammen enthält. Durch eine erste oder eine zweite Datenverarbeitungsstation, die Zugriff auf die Patientendaten sowie die erste Datenbank hat, werden durch Rückgriff auf die erste Datenbank und die Patientendaten ein oder mehrere Vorschläge zur Modifikation oder Beibehaltung des vom Patienten absolvierten Trainingsprogramms automatisch generiert und an der ersten oder einer weiteren Datenverarbeitungsstation ausgegeben. Die von den Patientendaten umfassten Messwerte bzw. Messgrößen können bspw. aus Protokollen der Trainingsdurchführung, bspw. Dauer, Art und Anzahl der Übungen, erreichte Punktzahlen aus den durchgeführten Übungen etc. stammen, die gemäß einer Ausführungsform des Verfahrens in elektronischer Form an die erste Datenverarbeitungsstation übermittelt werden oder von dieser aus einer oder mehreren entsprechenden Datenbanken abgerufen werden können. In gleicher Weise können Patientendaten aus einem Patiententagebuch oder einer elektronischen Patientenakte abgerufen werden. Neben den Messwerten von Messgrößen über die Durchführung und/oder die Zwischenergebnisse einzelner Trainingseinheiten umfassen die Patientendaten selbstverständlich auch andere Informationen, wie bspw. das Alter des Patienten oder eventuelle aktuelle oder Vorerkrankungen. Auf Basis der erfassten Patientendaten und des dem Patienten verordneten Trainingsprogramms werden durch Rückgriff auf die erste Datenbank durch die erste oder zweite Datenverarbeitungsstation entsprechend der dort gespeicherten Regeln automatisch ein oder mehrere Vorschläge zur Modifikation des verordneten Trainingsprogramms generiert und entweder direkt ausgegeben oder an eine separate Datenverarbeitungsstation des betreuenden Arztes oder Therapeuten übermittelt und dort ausgegeben. Der Arzt oder Therapeut kann dann ein oder mehrere dieser Vorschläge bestätigen, um das in einer Datenbank abgespeicherte, dem Patienten aktuell verordnete Trainingsprogramm entsprechend automatisch modifizieren zu lassen. Selbstverständlich kann sich bei Rückgriff auf die erste Datenbank auch ergeben, dass das aktuell verordnete Trainingsprogramm keiner Modifikation bedarf, so dass von der ersten oder zweiten Datenverarbeitungsstation in diesem Fall lediglich ein Hinweis zur Beibehaltung des Trainingsprogramms generiert wird.

Die Modifikation des Trainingsprogramms kann dabei eine Veränderung einer Vielzahl möglicher Konfigurationsparameter des Trainingsprogramms betreffen. Beispiele für derartige Konfigurationsparameter sind der Schwierigkeitsgrad einzelner Übungen, die Reihenfolge mehrerer aufeinanderfolgender Übungen, der Austausch von Übungseinheiten gegen andere Übungseinheiten, die Veränderung von Übungsdauern einzelner Übungen oder Teilübungen oder der Gesamtdauer des Trainingsprogramms oder die Verschiebung des Übungsschwerpunktes zwischen Teilübungen zu verschiedenen Fähigkeitsbereichen. Selbstverständlich ist dies keine abschließende Aufzählung, so dass alle für die Durchführung des Trainingsprogramms relevanten Parameter modifiziert werden können.

Durch das vorliegende Verfahren und das zugehörige System wird dem Arzt oder Therapeuten ein computergestütztes Hilfswerkzeug zur Verfügung gestellt, das in Abhängigkeit vom momentanen Stand des Trainingsprogramms bzw. der Entwicklung des Patienten während des Trainings automatisch das Training optimierende Modifizierungsvorschläge liefert, die der Arzt oder Therapeut dann lediglich noch bestätigen oder ablehnen muss. Er ist damit von der zeit- und arbeitsintensiven Bewertung der aktuellen Patientendaten und der eigenen Ableitung von Anderungsmaßnahmen entlastet.

Besonders vorteilhaft lässt sich das vorliegende Verfahren sowie das zugehörige System bei der telemedizinischen Betreuung von Patienten einsetzen. Bei der telemedizinischen Betreuung ist der Arzt oder Therapeut, der das Trainingsprogramm erstellt bzw. verordnet hat, bei der Durchführung der Übungen des Trainingsprogramms nicht persönlich anwesend. Somit muss er die Daten über die Durchführung des Trainings und den aktuellen Gesundheitsstatus bzw. Entwicklungsstand des Patienten, die er zu einer individuellen Optimierung des Trainingsprogramms benötigt, indirekt und nicht durch eigene Beobachtung bekommen.

Vorzugsweise wird bei dieser telemedizinisch betreuten Rehabilitation ein System mit Trainings-, Therapiekontroll-, Informations- und Kommunikationsfunktionen eingesetzt. Das System besteht aus mindestens einem Terminal für den Arzt oder Therapeuten, einem oder mehreren Patiententerminals und je nach Architektur optional aus einem zentralen oder dezentralen Server. Auf dem Terminal des Arztes oder Therapeuten, einer entsprechend ausgestalteten Datenverarbeitungsstation, erstellt der Arzt oder Therapeut individuell optimale Trainingsprogramme für den Patienten. Diese werden auf telematischem Weg zur (zweiten) Datenverarbeitungsstation, dem Patienten-Terminal, transferiert. Dort steht dem Patienten zur Durchführung der verschriebenen Trainingsübungen ein multimedialer Computerarbeitsplatz zur Verfügung. Bei der Durchführung des computerbasierten Trainings, vorzugsweise beim Patienten zu Hause, aber auch in ambulanten Einrichtungen, wie Selbsthilfegruppen oder in einer Rehaklinik, werden die Messgrößen direkt erfasst, die Aufschluss über den Umfang der Durchführung des Trainingsprogramms, bspw. Zeitdauer, Anzahl der Übungen oder Art der Übungen, und den Trainingserfolg, bspw. über erreichte Punktzahlen in einzelnen Übungen oder Einträge in ein Patiententagebuch, geben. Die Übertragung dieser Messwerte an den betreuenden Arzt oder Therapeuten stellt eine Feedback-Schleife dar, mittels derer das Trainingsprogramm kontinuierlich individuell optimiert werden kann.

Vorzugsweise werden die Messwerte der Messgrößen über die Durchführung des Trainings und/oder zur Quantifizierung von Zwischenergebnissen bzw. des bisherigen Trainingserfolgs an der zweiten Datenverarbeitungsstation bei der Durchführung des Trainingsprogramms automatisch erfasst oder durch den Patienten von Hand eingegeben. Je nach Durchführung des Verfahrens werden diese Messwerte über ein Netzwerk direkt an die erste Datenverarbeitungsstation übermittelt und/oder in einer Datenbank abgespeichert, auf die die erste Datenverarbeitungsstation Zugriff hat, oder die Vorschläge zur Modifikation des Trainingsprogramms direkt in der zweiten Datenverarbeitungsstation generiert und über das Netzwerk an die erste oder weitere Datenverarbeitungsstation übermittelt. Über das Netzwerk wird der zweiten Datenverarbeitungsstation in einer Ausführungsform des Verfahrens auch das verordnete oder modifizierte Trainingsprogramm übermittelt, das der Patient durchzuführen hat. Auf diese Weise lässt sich insbesondere bei einer telemedizinischen Betreuung, bei der die zweite Datenverarbeitungsstation im häuslichen Umfeld des Patienten steht, eine zeit- und arbeitssparende Betreuung durch den Arzt oder Therapeuten bei gleichzeitiger Optimierung des Trainingsprogramms durchführen.

Die Erfassung der Patientendaten erfolgt bei dem vorliegenden Verfahren vorzugsweise nicht nur einmalig sondern wiederholt während der Dauer des Trainingsprogramms, um rechtzeitig durch eine Modifikation des Trainingsprogramms auf eine veränderte oder unerwartete Entwicklung des Patienten reagieren zu können. Vorzugsweise wird zumindest ein Teil der Messwerte der jeweils erfassten Messgrößen der Patientendaten mit Schwellwerten verglichen, die bspw. vorab durch den behandelnden Arzt oder Therapeuten für die jeweilige Messgröße in einer Datenbank abgespeichert wurden. Durch diesen Vergleich kann festgestellt werden, ob der Trainingserfolg in einzelnen Übungssegmenten besser, schlechter oder gleich der Erwartung des Arztes oder Therapeuten ist. Bei einer Über- bzw. Unterschreitung der Schwellwerte kann dann die automatische Generierung eines Modifikationsvorschlages erfolgen. Neben der Festlegung der Schwellwerte gemäß der Erfahrung des Arztes oder Therapeuten können diese auch als Durchschnittswerte eines vergleichbaren Patientenkollektivs berechnet sein.

In letzterem Fall werden vorzugsweise Trainingsverlaufsdaten einer Vielzahl von zu einem Patientenkollektiv zusammengefassten Vergleichspatienten bereitgestellt, die ein vergleichbares Trainingsprogramm bereits durchgeführt haben. Aus diesen Trainingsverlaufsdaten werden mit Hilfe einer Berechnungsvorschrift eine oder mehrere Vergleichskurven für diejeweiligen Messgrößen berechnet und abgespeichert. Die jeweils aktuellen Messwerte des Patienten werden mit dem jeweiligen Zeitpunkt bzw. der jeweiligen Trainingsstufe entsprechenden Messwerten der abgespeicherten Vergleichskurve automatisch verglichen, um bei einer Abweichung der Messwerte des Patienten von der Vergleichskurve um einen vorgebbaren Mindestwert Vorschläge für eine Modifikation des Trainingsprogramms zu generieren. Die Trainingsverlaufsdaten sind hierbei vorzugsweise in einer Datenbank abgespeichert, auf die die erste Datenverarbeitungsstation Zugriff hat.

Bei einem Vorschlag für eine Modifikation des Trainingsprogramms können auch zusätzlich Hinweise generiert werden, die durch die Modifikation verursachte organisatorische Handlungsempfehlungen beinhalten. Derartige Handlungsempfehlungen können bspw. die Ressourcenplanung, wie bspw. Pflegedienstressourcen, die Überweisung des Patienten an andere Fachabteilungen oder externe Ärzte und Therapeuten oder eine Kostenträger-Information, wie bspw. die Bereitstellung eines Kostenübernahmeantrags oder einer Kostenabrechnung, umfassen.

In einer weiteren Ausbildung des vorliegenden Verfahrens können zusätzlich Verlaufsprognosen über die weitere Entwicklung des Patienten erstellt werden. So ist bspw. bei einem Patienten unmittelbar nach einem Schlaganfall häufig zu Beginn der Therapie nicht gleich vorhersagbar, ob der Therapieerfolg ausreichend für die Wiedereingliederung in das Berufsleben sein wird, oder ob die Berufsunfähigkeit trotz der Therapie fortbestehen wird. Durch wiederholte Erfassung der Messgrößen zur Quantifizierung der jeweils erreichten Zwischenergebnisse innerhalb des Trainingsprogramms kann eine Verlaufskurve des Therapiefortschritts erstellt und mit einer Vergleichskurve aus einem Patienten-Kollektiv mit Vergleichspatienten verglichen werden. Ein derartiger Vergleich kann auch differenziert durch Vergleich mit zwei disjunkten Untergruppen aus dem historischen Patienten-Kollektiv erfolgen, bei denen eine Untergruppe Patienten enthält, die berufsunfähig blieben, während die andere Untergruppe Patienten enthält, die wieder ins Berufsleben zurückkehren konnten. Durch Vergleich des Therapiefortschritts des Patienten mit den Vergleichskurven der beiden Untergruppen kann somit frühzeitig aus dem individuellen Therapieverlauf eine Schlussfolgerung gezogen werden, bspw. wenn die Verlaufskurve des Patienten den Bereich des berufsfähigen Kollektivs und auch den Bereich der Überschneidung beider Kollektive verlassen hat. In einem derartigen Fall kann wiederum automatisch eine entsprechende Information an den behandelnden Arzt oder Therapeuten gesendet werden, ggf. ergänzt mit Hinweisen auf Konsequenzen, wie bspw. einen Vorschlag für die Modifikation des Trainingsprogramms oder einen Hinweis auf die erforderliche Einleitung einer Berufsunfähigkeitsrente usw..

Selbstverständlich können die während des Trainingsprogramms erfassten Messgrößen auch die Compliance des Patienten umfassen, die ebenfalls für eine Modifikation des Trainingsprogramms ausschlaggebend sein kann.

Als besondere Variante der automatisierten Generierung von Vorschlägen zur Modifikation des Trainingsprogramms kann die Generierung eines Warnhinweises vorgesehen werden, der auf ungünstige Konfigurationsparameter des Trainingsprogramms verweist und mit Patientendaten verknüpft, die eine Begründung für den Warnhinweis darstellen.

Die in der ersten Datenbank enthaltenen Regeln berücksichtigen insbesondere auch in der Medizin bekannte Einflüsse von anderen Erkrankungen (Co-Morbidität, Multimorbidität), die Einschränkungen für Trainingsprogramme zu der gerade behandelten Grunderkrankung darstellen. So muss bspw. die Kreislaufbelastung durch ein physio-therapeutisches Training nach einem Gelenkimplantat bei vorliegender Herzinsuffizienz des Patienten anders gewählt werden als bei herzgesunden Patienten. Die Information über Multimorbiditäten kann bspw. automatisch aus einer elektronischen Patientenakte des Patienten entnommen werden. Dies hat den großen Vorteil, dass auch plötzlich neu auftretende Erkrankungen des Patienten, die der telemedizinisch behandelnde Arzt evtl. gar nicht erfahren würde, durch die wiederholte Erfassung der Patientendaten während der Therapie automatisch berücksichtigt werden und zu einer adäquaten Modifikation des Trainingsprogramms führen.

In einer Weiterbildung des vorliegenden Verfahrens können weitere Daten über den Therapieerfolg als Input für die Generierung von Modifikationsvorschlägen dadurch gewonnen werden, dass zusätzlich zu den Trainingseinheiten in regelmäßigen Abständen Testprogramme durchgeführt werden, die den Therapieerfolg messen. Mit diesem zusätzlichen Staging-Tests können Training und Messung des Erfolgs unabhängig voneinander durchgeführt werden.

Weiterhin berücksichtigt das vorliegende Verfahren vorzugsweise gegenseitige Abhängigkeiten beim Trainingserfolg in unterschiedlichen Fähigkeitskategorien, wenn der Patient gleichzeitig an mehreren Fähigkeitsdefiziten leidet. Hierbei kann bei der automatischen Generierung von Modifikationsvorschlägen durch entsprechend vorgesehene Regeln in der ersten Datenbank darauf geachtet werden, dass ein ausgewogener Trainingserfolg in allen Kategorien eintritt, und nicht eine Kategorie sich zu Lasten einer anderen verbessert. Fähigkeitskategorien, die in beliebiger Kombination gemeinsam trainiert werden können, sind bspw. linguistische Störungen, Koordinationsstörungen, Gleichgewichtsstörungen, Reaktionsstörungen, kognitive Störungen, sprachmotorische Störungen, Erinnerungsstörungen etc..

Das vorliegende System zur Unterstützung der Therapieplanung umfasst zumindest eine erste Datenverarbeitungsstation sowie eine zweite Datenverarbeitungsstation, die zumindest zeitweise über ein Netzwerk miteinander Daten austauschen können. Die erste oder zweite Datenverarbeitungsstation ist mit einer ersten Datenbank verbunden, die Regeln zur Verknüpfung von Patientendaten mit Vorschlägen zur Vorgabe und/oder Modifikation von Trainingsprogrammen enthält. Die erste oder die zweite Datenverarbeitungsstation umfasst ein erstes Modul zur automatischen Generierung von Vorschlägen zur Modifikation eines Trainingsprogramms für den Patienten durch Rückgriff auf die erste Datenbank sowie zur Ausgabe der Vorschläge oder zur Übermittlung der Vorschläge an die erste bzw. eine weitere Datenverarbeitungsstation. Die zweite Datenverarbeitungsstation umfasst ein zweites Modul zur Erfassung von Messwerten von Messgrößen über die Durchführung und/oder zur Quantifizierung von Zwischenergebnissen eines vom Patienten absolvierten Trainingsprogramms sowie zur Übertragung dieser Messwerte an die erste Datenverarbeitungsstation und/oder zur Speicherung dieser Messwerte in einer zweiten Datenbank oder zur Übermittlung bereits generierter Vorschläge an die erste oder weitere Datenverarbeitungsstation. Die zweite Datenverarbeitungsstation ist hierbei vorzugsweise über ein oder mehrere Schnittstellen mit entsprechenden Messwertaufnehmern verbunden und/oder umfasst Module zum computergestützten Test des Patienten.

Die erste oder zweite Datenverarbeitungsstation mit dem ersten Modul sowie der ersten Datenbank stellt somit ein Expertensystem dar, das die Patientendaten bewertet und daraus Vorschläge für eine optimierende Modifikation des Trainingsprogramms ableitet und ausgibt bzw. an den Arzt oder Therapeuten, insbesondere dessen Datenverarbeitungsstation, übermittelt. Die erste Datenverarbeitungsstation kann dabei ein Server oder auch die Datenverarbeitungsstation des Arztes oder Therapeuten sein. Das Expertensystem hat hierbei vorzugsweise Zugriff auf eine Datenbank mit der elektronischen Patientenakte sowie ggf. auf eine weitere Datenbank mit den im Rahmen des Trainingsprogramms erfassten Messgrößen.

Das vorliegende Verfahren sowie das zugehörige System werden nachfolgend anhand eines Ausführungsbeispiels in Verbindungmit den Zeichnungen ohne Beschränkung des allgemeinen Erfindungsgedankens nochmals erläutert. Hierbei zeigen:
- Fig. 1: einen Überblick über das Verfahren bzw. das System zur Unterstützung der Therapieplanung gemäß der vorliegenden Erfindung; und
- Fig. 2: einen Überblick über die Vernetzung der ersten und zweiten Datenverarbeitungsstation inkl. der Erfassung einzelner Messgrößen.

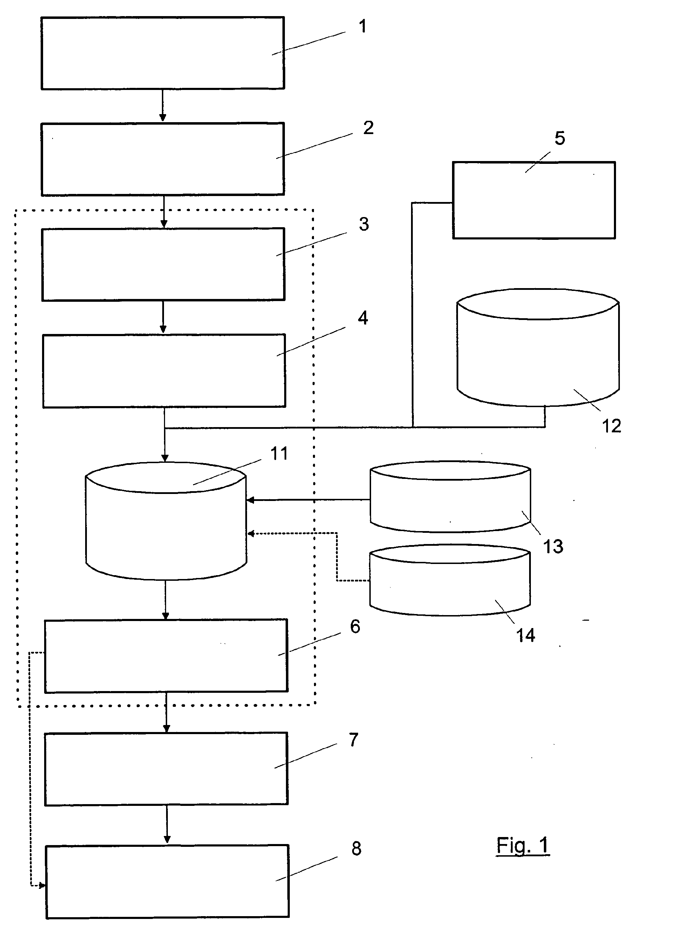

Figur 1 zeigt einen schematischen Ablauf bei der Durchführung des vorliegenden Verfahrens sowie einen Teil des zugehörigen Systems. Im ersten Schritt 1 wird für den Patienten ein individuelles Trainingsprogramm erstellt und im Schritt 2 an den Computer-Arbeitsplatz (zweite Datenverarbeitungsstation) des Patienten über eine entsprechende Netzwerkverbindung übermittelt. Während der Dauer des Trainingsprogramms werden wiederholt relevante Messgrößen bei der Durchführung des Trainings erfasst (Schritt 3) und an das Expertensystem, insbesondere die erste Datenverarbeitungsstation mit dem entsprechenden Verarbeitungsmodul übersendet (Schritt 4). Weiterhin werden Daten aus einer Datenbank 12 mit der elektronischen Patientenakte des Patienten abgefragt sowie ggf. weitere Informationen durch Befragung des Patienten und digitale Erfassung der entsprechenden Information (Schritt 5).

Durch Rückgriff auf eine Datenbank 11 mit Expertenregeln zur Optimierung eines Trainingsprogramms werden schließlich automatisch Vorschläge zur Modifikation des dem Patienten verordneten Trainingsprogramms generiert (Schritt 6). Es versteht sich von selbst, dass entsprechende Modifikations-Vorschläge nur bei Abweichung des aktuell verordneten Trainingsprogramms von den in der Datenbank 11 enthaltenen Verknüpfungen generiert werden. Vorzugsweise wird ein Modifikations-Vorschlag nur dann erstellt, wenn die jeweils erfassten Messgrößen de-s Patienten entsprechende Schwellwerte über- oder unterschreiten, die in einer weiteren Datenbank 13 abgespeichert sind, auf die das Expertensystem Zugriff hat.

Die Vorschläge zur Modifikation des Trainingsprogramms werden schließlich an der ersten Datenverarbeitungsstation dargestellt oder, falls dem Arzt oder Therapeuten eine andere Datenverarbeitungsstation zugeordnet ist, an diese Datenverarbeitungsstation über ein Netzwerk übermittelt und dort entsprechend ausgegeben (Schritt 7). Bei Bestätigung eines oder mehrerer der Vorschläge für die Modifikation des Trainingsprogramms wird das entsprechend modifizierte Trainingsprogramm schließlich an die (zweite) Datenverarbeitungsstation des Patienten übermittelt, um das bisher verordnete Trainingsprogramm zu ersetzen (Schritt 8).

Als Beispiel für eine erforderliche Modifikation des Trainingsprogramms in der kardiologischen Rehabilitation wird ein Herzinfarktpatient angenommen, der nach dem Aufenthalt in der Klinik sein als Trainingsprogramm verordnetes Ausdauertraining am Fahrradergometer zu Hause fortsetzt. Dem Patienten wird hierbei eine (zweite) Datenverarbeitungsstation zur Verfügung gestellt, die zeitweise mit einer (ersten) Datenverarbeitungsstation in der Klinik über ein Netzwerk Daten austauschen kann. Während des Trainings werden Vitalparameter wie Herzfrequenz und Blutdruck gemessen, nach dem Training über die zweite Datenverarbeitungsstation vom Patienten Aspekte der Befindlichkeit wie Schmerz und Anstrengung abgefragt. Die entsprechenden Werte der Vitalparameter sowie der Befindlichkeit werden über das Netzwerk an die erste Datenverarbeitungsstation in der Klinik übermittelt. Die Messungen ergeben beispielsweise hohe (schlechte) Werte, die Befragung dagegen geringe (gute) Werte - der Patient hat das Training als kaum schmerzhaft und wenig anstrengend empfunden. In diesem Beispiel könnte die automatisierte Datenauswertung durch die erste Datenverarbeitungsstation in der Klinik folgende Vorschläge für eine Multiplikation des Trainings-Programms fürdie weitere Behandlung generieren:
- 1. Modifikation des Trainings: Da die objektiven Daten unter Berücksichtigung der vorliegenden Vorerkrankung, bspw. Arteriosklerose der Koronargefäße, ungünstig sind, wird die Trainingsintensität, bspw. angegeben in Watt, verringert, die Trainingsdauer erhöht und die Trainingsdichte beibehalten. Die Information über das Vorliegen von Arteriosklerose geht aus der elektronischen Patientenakte hervor, die gleichzeitig abgeglichen wird. Die subjektiven Daten (Patiententagebuch) gehen in die Erstellung des neuen Trainings ein, spielen aber eine untergeordnete Rolle. Alternativ könnte ein Wechsel der Belastungsform sowie eine Anpassung der Belastungskomponenten vorgeschlagen werden, bspw. durch Austausch des Fahrradergometertrainings durch Walking.
- 2. Modifikation des flankierenden Monitorings: Die Zahl der während des Trainings und nach dem Training erfolgenden Messungen wird erhöht.
- 3. Information und Kommunikation: Dem Therapeuten wird vorgeschlagen, mit dem Patienten Kontakt aufzunehmen. Der Patient wird über die Veränderungen umfassend informiert.

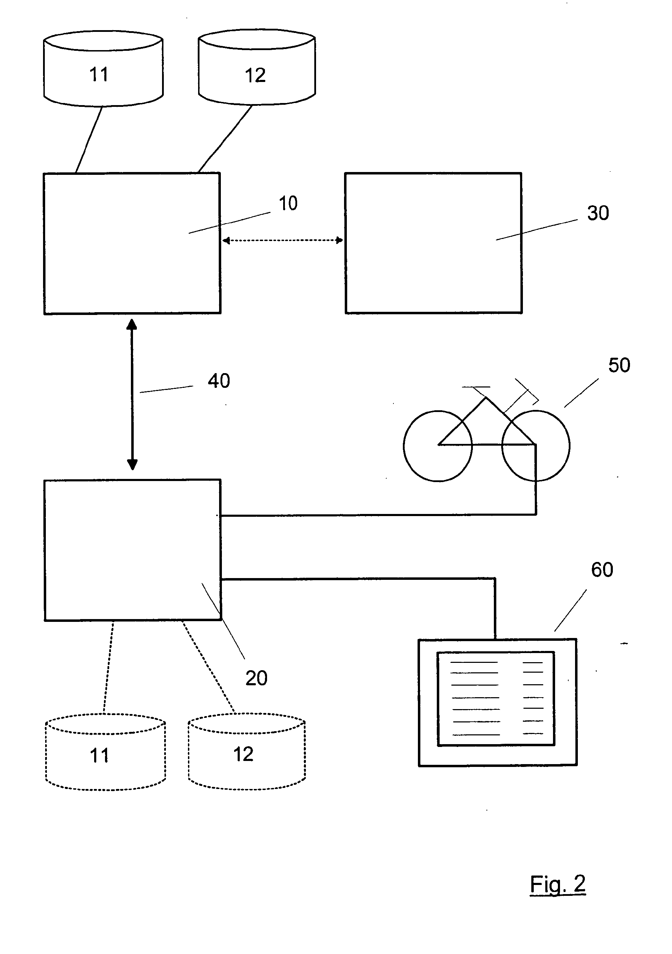

Figur 2 zeigt beispielhaft die Vernetzung der ersten Datenverarbeitungsstation 10 mit der zweiten Datenverarbeitungsstation 20 im häuslichen Umfeld des Patienten über ein Netzwerk 40. Die erste Datenverarbeitungsstation 10 kann über das gleiche oder ein anderes Netzwerk 40 mit einer weiteren Datenverarbeitungsstation 30 verbunden sein, auf die der Arzt oder Therapeut Zugriff hat. Die erste Datenverarbeitungsstation 10 hat insbesondere Zugriff auf eine Datenbank 12 mit der elektronischen Patientenakte sowie eine Datenbank 11 mit den Expertenregeln. In einer anderen Ausgestaltung kann auch die zweite Datenverarbeitungsstation 20 das Expertensystem mit Zugriff auf die ensprechenden Datenbanken 11, 12 darstellen (gestrichelt angedeutet).

Die Erfassung der jeweils aktuellen Messwerte der Messgrößen über die Durchführung und/oder die Zwischenergebnisse des vom Patienten absolvierten Trainingsprogramms kann über entsprechende Schnittstellen bspw. direkt von einem Ergometer 50 erfasst werden oder, im Falle von entsprechend durch den Patienten auszuwertenden Fragebögen, die am Bildschirm 60 des Computerarbeitsplatzes dargestellt werden, direkt über die zweite Datenverarbeitungsstation 20 erfolgen.

## Patentansprüche

1. Verfahren zur Unterstützung der Therapieplanung in der Rehabilitation eines Patienten, bei dem:
- Patientendaten eines Patienten erfasst werden, die Messwerte von Messgrößen über die Durchführung und/oder zur Quantifizierung von Zwischenergebnissen eines vom Patienten absolvierten Trainingsprogramms umfassen;
- eine erste Datenbank (11) bereitgestellt wird, die Regeln zur Verknüpfung von Patientendaten mit Vorschlägen zur Modifikation von Trainingsprogrammen enthält;
- von einer ersten oder zweiten Datenverarbeitungsstation (10, 20) automatisch ein oder mehrere Vorschläge zur Modifikation oder Beibehaltung des vom Patienten absolvierten Trainingsprogramms durch Rückgriff auf die erste Datenbank (11) und die Patientendaten generiert werden; und
- die generierten Vorschläge an der ersten (10) oder einer weiteren Datenverarbeitungsstation (30) ausgegeben werden.

2. Verfahren nach Anspruch 1,
**dadurch gekennzeichnet,**
**dass** zumindest ein Teil der Patientendaten von einer zweiten Datenbank (12) abgerufen wird, die eine elektronische Patientenakte enthält.

3. Verfahren nach Anspruch 1 oder 2,
**dadurch gekennzeichnet,**
**dass** die Messwerte der Messgrößen über die Durchführung und/oder zur Quantifizierung von Zwischenergebnissen des vom Patienten absolvierten Trainingsprogramms an der zweiten Datenverarbeitungsstation (20) bei der Durchführung des Trainingsprogramms automatisch erfasst oder vom Patienten eingegeben werden.

4. Verfahren nach Anspruch 3,
**dadurch gekennzeichnet,**
**dass** die erfassten oder eingegebenen Messwerte über ein Netzwerk (40) direkt an die erste Datenverarbeitungsstation (10) übermittelt und/oder in einer zweiten Datenbank (12) gespeichert werden.

5. Verfahren nach Anspruch 3 oder 4,
**dadurch gekennzeichnet,**
**dass** ein oder mehrere der generierten Vorschläge durch eine Eingabe eines Benutzers bestätigt und das abgespeicherte Trainingsprogramm des Patienten mit diesen bestätigten Vorschlägen modifiziert wird.

6. Verfahren nach Anspruch 5,
**dadurch gekennzeichnet,**
**dass** das modifizierte Trainingsprogramm über ein Netzwerk (40) an die zweite Datenverarbeitungsstation (20) übermittelt wird, um das dort gespeicherte Trainingsprogramm zu ersetzen.

7. Verfahren nach einem der Ansprüche 1 bis 6,
**dadurch gekennzeichnet,**
**dass** die Patientendaten wiederholt während der Dauer des Trainingsprogramms erfasst und aktualisiert werden, um automatisch Vorschläge zur Modifikation des Trainingsprogramms zu generieren.

8. Verfahren nach einem der Ansprüche 1 bis 7,
**dadurch gekennzeichnet,**
**dass** die Messwerte automatisch mit Schwellwerten aus einer dritten Datenbank (13) verglichen werden, um bei Unter- bzw. Überschreiten der Schwellwerte einen oder mehrere Vorschläge zur Modifikation des Trainingsprogramms zu generieren.

9. Verfahren nach einem der Ansprüche 1 bis 7,
**dadurch gekennzeichnet,**
**dass** Trainingsverlaufsdaten einer Vielzahl von zu einem Patientenkollektiv zusammengefassten Vergleichs-Patienten bereitgestellt werden, die ein vergleichbares Trainingsprogramm bereits durchgeführt haben, aus den Trainingsverlaufsdaten des Patienten-Kollektivs mit Hilfe einer Berechnungsvorschrift ein oder mehrere Vergleichskurven für die Messgrößen berechnet und abgespeichert werden, die jeweils aktuellen Messwerte des Patienten mit dem jeweiligen Zeitpunkt bzw. der jeweiligen Trainingsstufe entsprechenden Messwerten der abgespeicherten Vergleichskurve automatisch verglichen werden, um bei Abweichung der Messwerte des Patienten von der Verlaufskurve um einen vorgebbaren Mindestwert Vorschläge für eine Modifikation des Trainingsprogramms zu generieren.

10. Verfahren nach Anspruch 9,
**dadurch gekennzeichnet,**
**dass** Vergleichskurven für zumindest zwei disjunkte Untergruppen von Vergleichs-Patienten berechnet und für den Vergleich mit den aktuellen Messwerten des Patienten herangezogen werden, um die Zugehörigkeit des Patienten zu einer der Untergruppen zu bestimmen.

11. Verfahren nach Anspruch 9 oder 10,
**dadurch gekennzeichnet,**
**dass** das Bereitstellen der Trainingsverlaufsdaten einer Vielzahl von Vergleichs-Patienten durch Zugriff auf eine vierte Datenbank (14) erfolgt, die diese Trainingsverlaufsdaten enthält.

12. Verfahren nach einem der Ansprüche 1 bis 11,
**dadurch gekennzeichnet,**
**dass** bei Erkennung einer ungünstigen Verknüpfung des Trainingsprogramms des Patienten mit den erfassten Patientendaten automatisch ein Warnhinweis erzeugt und zusammen mit dem für die ungünstige Verknüpfung betreffenden Teil der Patientendaten an der ersten (10) und/oder weiteren Datenverarbeitungsstation (30) ausgegeben wird.

13. Verfahren nach einem der Ansprüche 1 bis 12,
**dadurch gekennzeichnet,**
**dass** bei der Generierung eines Vorschlags für eine Modifikation des Trainingsprogramms zusätzlich Hinweise generiert und ausgegeben werden, die durch die Modifikation verursachte organisatorische Handlungsempfehlungen beinhalten.

14. Verfahren nach einem der Ansprüche 1 bis 13,
**dadurch gekennzeichnet,**
**dass** die in der ersten Datenbank (11) enthaltenen Regeln Einflüsse von anderen Krankheiten des Patienten, die in den Patientendaten aufgenommen sind, auf das Trainingsprogramm berücksichtigen.

15. Verfahren nach einem der Ansprüche 1 bis 14,
**dadurch gekennzeichnet,**
**dass** einige der Messwerte aus Staging-Tests erhalten werden, die parallel zum Trainingprogramm durchgeführt werden.

16. Verfahren nach einem der Ansprüche 1 bis 15,
**dadurch gekennzeichnet,**
**dass** die in der ersten Datenbank (11) enthaltenen Regeln gegenseitige Abhängigkeiten beim Trainingserfolg in unterschiedlichen Fähigkeitskategorien berücksichtigt, um einen ausgewogenen Trainingserfolg zu gewährleisten.

17. Verfahren nach einem der Ansprüche 1 bis 16,
**dadurch gekennzeichnet,**
**dass** die Therapie als telemedizinische Behandlung durchgeführt wird, wobei die zweite Datenverarbeitungsstation (20) im häuslichen Umfeld des Patienten steht.

18. System zur Unterstützung der Therapieplanung in der Rehabilitation eines Patienten mit zumindest einer ersten Datenverarbeitungsstation (10) sowie einer zweiten Datenverarbeitungsstation (20), die zumindest zeitweise über ein Netzwerk (40) miteinander Daten austauschen können,
wobei die erste oder die zweite Datenverarbeitungsstation (10) mit einer ersten Datenbank (11) verbunden ist, die Regeln zur Verknüpfung von Patientendaten mit Vorschlägen zur Vorgabe und/oder Modifikation von Trainingsprogrammen enthält, und ein erstes Modul zur automatischen Generierung von Vorschlägen zur Vorgabe und/oder Modifikation eines Trainingsprogramms für den Patienten durch Rückgriff auf die erste Datenbank (11) sowie zur Ausgabe der Vorschläge oder zur Übermittlung der Vorschläge an die erste (10) bzw. eine weitere Datenverarbeitungsstation (30) umfasst, und
wobei die zweite Datenverarbeitungsstation (20) ein zweites Modul zur Erfassung der Messwerte von Messgrößen über die Durchführung und/oder zur Quantifizierung von Zwischenergebnissen eines vom Patienten absolvierten Trainingsprogramms und zur Übertragung dieser Messwerte oder bereits generierter Vorschläge an die erste Datenverarbeitungsstation (10) und/oder zur Abspeicherung dieser Messwerte in einer zweiten Datenbank (12) umfasst.

19. System nach Anspruch 18,
**dadurch gekennzeichnet,**
**dass** die zweite Datenverarbeitungsstation (20) über eine oder mehrere Schnittstellen mit Messwertaufnehmern zur Erfassung zumindest eines Teils der Messwerte verbunden ist und/oder Module zum computergestützten Test des Patienten umfasst.

20. System nach Anspruch 18 oder 19,
**dadurch gekennzeichnet,**
**dass** die in der ersten Datenbank (11) enthaltenen Regeln Einflüsse von anderen Krankheiten des Patienten berücksichtigen, die in den Patientendaten enthalten sind.

21. System nach einem der Ansprüche 18 bis 20,
**dadurch gekennzeichnet,**
**dass** die in der ersten Datenbank (11) enthaltenen Regeln gegenseitige Abhängigkeiten beim Trainingserfolg in unterschiedlichen Fähigkeitskategorien berücksichtigen, um einen ausgewogenen Trainingserfolg zu gewährleisten.

22. System nach einem der Ansprüche 18 bis 21,
**dadurch gekennzeichnet,**
**dass** die erste oder zweite Datenverarbeitungsstation (10) mit einer dritten Datenbank (13) verbunden ist, die Schwellwerte für die Messgrößen enthält, und das erste Modul zum automatischen Vergleich der Messwerte des Patienten mit den Schwellwerten ausgebildet ist, um bei Unter- bzw. Überschreiten der Schwellwerte einen oder mehrere Vorschläge zur Modifikation des Trainingsprogramms zu generieren.

23. System nach einem der Ansprüche 18 bis 21,
**dadurch gekennzeichnet,**
**dass** die erste oder zweite Datenverarbeitungsstation (10) mit einer vierten Datenbank (14) verbunden ist, die Trainingsverlaufsdaten einer Vielzahl von zu einem Patienten-Kollektiv zusammengefassten Vergleichs-Patienten enthält, und das erste Modul zur Bildung und Speicherung einer Trainings-Verlaufskurve aus den Trainingsverlaufsdaten des Patienten-Kollektivs und zum automatischen Vergleich der Messwerte des Patienten mit der Trainings-Vergleichskurve ausgebildet ist, um bei einer Abweichung um einen vorgebbaren Mindestwert Vorschläge zur Modifikation des Trainingsprogramms zu generieren.
